(19) Europäisches Patentamt

European Patent Office

Office européen des brevets

(11) **EP 2 737 943 A2**

(12) # EUROPEAN PATENT APPLICATION
published in accordance with Art. 153(4) EPC

(43) Date of publication:
04.06.2014 Bulletin 2014/23

(21) Application number: 12819876.9

(22) Date of filing: 30.07.2012

(51) Int Cl.:
**B01J 27/188** (2006.01)

(86) International application number:
**PCT/JP2012/069305**

(87) International publication number:
**WO 2013/018752 (07.02.2013 Gazette 2013/06)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: 29.07.2011 JP 2011167366

(71) Applicant: **Nippon Kayaku Kabushiki Kaisha
Tokyo 102-8172 (JP)**

(72) Inventors:
• **OKUMURA, Kimito
SanyoOnoda-shi
Yamaguchi 757-8686 (JP)**

• **KOBAYASHI, Yasushi
SanyoOnoda-shi
Yamaguchi 757-8686 (JP)**
• **HIRAOKA, Ryota
Sanyo Onoda-shi
Yamaguchi 757-8686 (JP)**
• **DUBOIS, Jean-Luc
69390 Millery (FR)**

(74) Representative: **Gille Hrabal
Brucknerstrasse 20
40593 Düsseldorf (DE)**

(54) **CATALYST FOR MANUFACTURE OF ACROLEIN AND ACRYLIC ACID BY MEANS OF DEHYDRATION OF GLYCERIN, AND MANUFACTURING METHOD FOR SAME**

(57)     An object of the present invention is to provide a catalyst for glycerin dehydration reaction for producing unsaturated aldehyde and unsaturated carboxylic acid at higher yield by a dehydration reaction of glycerin, and that can reduce a decrease in time of the conversion ratio of glycerin and the yields of unsaturated aldehyde and of unsaturated carboxylic acid. Another object of the present invention is to provide a catalyst for glycerin dehydration reaction that can produce acrolein and acrylic acid at higher yield by the dehydration reaction of glycerin, and the catalyst has a longer life. Still another object of the present invention is to provide a method for preparing the catalysts above. A catalyst for glycerin dehydration comprising a carrier, wherein the carrier includes at least one metal oxide selected from the group consisting $TiO_2$, $SiO_2$, $Al_2O_3$, $ZrO_2$, and $Nb_2O_5$, wherein the carrier includes a bimodal porous structure in which the volume ratio between a pore volume of mesopores having a pore size of more than 2 nm to less than 50 nm to a pore volume of macropores having a pore size of 50 nm or more is equal to or more than 0.5, and wherein the carrier configures to support a W-containing metal oxide thereon or a metal oxide containing at least one element selected from the group consisting of P, Si, Mo, and V, in addition to the W-containing metal oxide thereon, and to cause a catalytic dehydrogenation reaction with glycerin to produce acrolein and acrylic acid.

EP 2 737 943 A2

**Description**

[Technical Field]

This invention relates to a novel dehydration catalyst. In particular, this invention relates to a novel catalyst for preparing acrolein and acrylic acid by catalytic dehydration reaction of glycerin in gas phase or liquid phase, and a process for preparing the catalyst.

[Background]

Acrolein is used in production of methionine and is a derivative for producing amino acid used as an animal feed supplement, which has emerged as a substitute for fooder. Acrolein is a synthetic intermediate of acrylic acid and leads, via reaction with methyl vinyl ether then hydrolysis, to glutaraldehyde or other pyridines, which has many uses in leather tanning.

Acrylic acid is a material used in a variety of industrial products and is an important compound used for industrial polymerization of a monomer and comonomer to produce a final product such as polyacrylates and polyacrylamide prepared by polymerization of acrylic acid or its derivatives. One of the important applications of acrylic acid is high water-absorption resins prepared by partial neutralization of a mixture of acrylic acid and sodium acrylate or other cation. In practice, acrylic acid is polymerized and the resulting polyacrylic acid is partly neutralized. These polymers or copolymer are utilized widely in various fields such as sanitation, detergent, coating material, varnish, adhesive, paper, fabric and leather.

Acrolein and acrylic acid are currently produced in industrial scale by a process for oxidizing propylene by using a catalyst in the presence of oxygen. Generally, this reaction is carried in gas phase. Acrylic acid is usually produced by two step reactions. In the first step, acrolein-rich product is prepared from propylene but little acrylic acid is produced in this stage. Acrylic acid is obtained by a selective oxidation of acrolein in the second step.

The two step reaction is carried out in multi-tubular reactors under two different reaction conditions and catalysts each suitable for each reactor. There is no necessity to effect purification of acrolein obtained in the first step. The oxidation reaction of propylene to acrolein and acrylic acid is carried out in a fixed bed reactor, unlike many other selective oxidation processes.

A starting material used in production of acrolein and acrylic acid is derived from petroleum and natural gas which are no regenerable fossil resources. However, it has been very important to produce them from renewable sources to reduce the global warming gas. Such change is the responsibility of industry major powers and can contribute to relaxation of the environmental loads and reduction of global warming gas.

Glycerin is obtained as a by-product when bio-diesel fuel is produced, and is considered as one of feedstock for propylene alternative and acrolein can be produced by catalytic dehydration reaction of glycerin. This process route responds to the concept of green chemistry for the environmental protection. Glycerin can be reduced by several processes such as fermentation and hydrogenolysis of sugar.

This process route is similar to the propylene oxidation process, since acrolein is prepared in the first step and the second step is carried out in the same reaction condition as the first step. Indeed the first step is however different from the usual propylene oxidation process. In fact, a solid catalyst which is different from those used in the propylene oxidation is used in the dehydration reaction in gas phase, and in this process, much water together with acrolein-rich gas is supplied to the second step for producing acrylic acid. Still more, a composition of by-products is very different due to completely different reaction mechanism.

Many catalysts have already been reported for producing acrolein by the glycerin dehydration reaction.

Patent Document 1 (US 5,387,720) discloses a process for producing acrolein by glycerin dehydration on a variety of solid acids in gas phase and liquid phase. Hammett index is defined as +2 or less, more preferably -3 or less, so that corresponding catalyst is for example natural or synthetic silicon compounds such as mordenite, montmorillonite, zeolite, monobasic acids, dibasic acid, tribasic acids supported on alumina and titania; 綱 -alumina, $ZnO/Al_2O_3$, or heteropolyacids. It is believed that a problem shown in FR 695,931 of by-products which are generated in case of an iron phosphate catalyst system can be solved.

Patent Document 2 (WO2006/087084) teaches that a strong solid acid having Hammett index from -9 to -18 permits to produce acrolein at high activity by glycerin dehydration and to suppress degradation of catalyst.

Patent Document 3 (WO2009/044081) discloses a glycerin dehydration reaction effected in the presence of a catalyst containing oxygen, iron, phosphorus, and alkali metal, and one or more elements selected from alkali-earth metals (Al, Si, B, Co, Cr, Ni, V, Zn, Zr, Sn, Sb, Ag, Cu, Nb, Mo, Y, Mn, Pt, and Rh) and rare earths (La, Ce, and Sm).

Patent Document 4 (WO2009/128555) discloses a process for preparing acrolein comprising a glycerin dehydration reaction effected in the presence of a catalyst consisting of a compound in which protons in heteropolyacid are exchanged with more than one cation of elements selected from a group consisting of Group 1 to Group 16 of the periodic table.

Patent Document 5 (WO2010/046227) discloses a glycerin dehydration reaction effected in the presence of a catalyst containing oxygen, phosphorus, and at least one element selected from a group consisting of vanadium, boron, and aluminum.

The conventional catalysts proposed in these prior documents, however, have certain problems such as a deposition of cokes which degrade the catalyst, and a development of secondary reactions such as an addition reaction of acrolein to hydroxy acetone, propionaldehyde, acetaldehyde, acetone, or glycerin, a polycondensation of glycerin, a formation of by-products such as cyclic glycerin ether, phenol, and poly aromatic compounds.

Therefore, to reduce generation of coke and degradation of catalysis and to suppress formation of by-products, such much detailed technology that physical properties of catalyst are limited as well as desirable catalytic active species are limited has been proposed.

Patent Document 6 (WO2007/058221) and Patent Document 7 (JP-A-2008-088149) disclose that an element of the Group 6 (tungsten, chromium, or molybdenum) or heteropolyacid is impregnated on an inorganic carrier containing at least one element selected from silicon, aluminum, titanium, and zirconium. The documents disclose in their claims that the inorganic carrier has a bimodal porous structure. The yield is improved when heteropolyacid is supported on a bipore silicon oxide carrier and the yield of higher than 80% is obtained comparing to a monopore silicon oxide carrier. However, the concentration of aqueous solution of glycerol is not high, and the proportion of hydroxy acetone which may be formed by dehydration of glycerin terminal is high. The prior documents disclose in their preferred examples that a carrier supporting heteropolyacid had the specific surface area of 30 to 1500 $m^2$/g, bimodal pore structure, macro pore of 0.5 to 200 m, nanopore of 1 to 50 nm, as well as a pore volume of 0.3 to 4 $cm^3$/g.

Patent Document 8 (JP-A-2010-253374) discloses that a bipore silicon oxide carrier supporting boron phosphate thereon can prevent a decrease of the yield of acrolein in time in comparison to a monopore silicon oxide carrier. The active species to be supported are at least one element selected from Al, Zr, and B, and/or crystalline salt of at least one element selected from rare earth elements and phosphorus. Its physical properties are characterized in that the carrier contains 90 mass % of silica, macropore of 0.1 to 30 · m, nanopore of 1 to 50 nm, and the carrier is amorphous. There is no detailed limitation of a pore volume of bipore and a relation between a volumetric basis mode diameter and catalytic behavior of the bipore carrier.

Patent Document 9 (JP-A-2007-301506) reports that the conversion of glycerin and the yield of acrolein in catalytic glycerin dehydration can be improved by using a molded catalyst of crystalline metallosilicate having a ratio of Si/T (T = Al, B, Ti, Cu, In, Cr, Fe, Co, Ni, Zn, or Ga) of less than 3000, a volumetric basis mode diameter of the molded catalyst measured by the mercury press-in method being smaller than 0.8 · m. This document describes primary and secondary pores of the metallosilicate, but is silent on a pore volume and volume ratio of bipores.

As explained above, the prior documents merely disclose some catalytic active species having limited physical parameters, such as a catalyst supporting the Group 6 element; a catalyst supporting heteropolyacid; a catalyst supporting crystalline salt of Al, Zr, B, and phosphorus; and metallosilicate. The prior documents also disclose some physical property parameters such as the specific surface area, a range of bipore diameters, and a cumulative pore volume of bipores. However the prior documents are silent on each pore volume of bipores and any pore volume ratios between mesopore and macropore.

[Citation List]

[Patent Literature]

[Patent Document 1] US 5,387,720
[Patent Document 2] WO2006/087084
[Patent Document 3] WO2009/044081
[Patent Document 4] WO2009/128555
[Patent Document 5] WO2010/046227
[Patent Document 6] WO2007/058221
[Patent Document 7] JP-A-2008-088149
[Patent Document 8] JP-A-2010-253374
[Patent Document 9] JP-A-2007-301506

[Summary of Invention]

[Technical Problem]

An object of the present invention is to provide a catalyst for glycerin dehydration reaction for producing unsaturated aldehyde and unsaturated carboxylic acid at higher yield by a dehydration reaction of glycerin, and that can reduce a decrease in time of the conversion ratio of glycerin and the yields of unsaturated aldehyde and of unsaturated carboxylic acid.

Another object of the present invention is to provide a catalyst for glycerin dehydration reaction that can produce acrolein and acrylic acid at higher yield by the dehydration reaction of glycerin, and the catalyst has a longer life.

Still another object of the present invention is to provide a method for preparing the catalysts above.

[Solution to Problem]

The present invention thus provides a catalyst for producing unsaturated aldehyde and unsaturated carboxylic acid, especially acrolein and acrylic acid, by using glycerin which is not derived from petroleum, at higher yield and with preventing decrease of the yield in time. The present invention also provides a method for preparing the catalyst.

The present inventors have developed, in order to the solve the above problems, a catalyst including at least one metal oxide selected from the group consisting $TiO_2$, $SiO_2$, $Al_2O_3$, $ZrO_2$, and $Nb_2O_5$, wherein the carrier includes a bimodal porous structure in which, as measured by the mercury press-in method, the volume ratio between a pore volume of mesopores having a pore size of more than 2 nm to less than 50 nm to a pore volume of macropores having a pore size of 50 nm or more is equal to or more than 0.5, and wherein the carrier configures to support a W-containing (tungsten-containing) metal oxide thereon or a metal oxide containing at least one element selected from the group consisting of P, Si, Mo, and V, in addition to the W-containing metal oxide thereon, and to cause a catalytic dehydrogenation reaction with glycerin to produce acrolein and acrylic acid. The present inventors have found that the catalyst above produces unsaturated aldehyde and unsaturated carboxylic acid by a catalytic dehydration reaction of glycerin for a longer time duration at higher yield, and have completed the present invention. The present invention has the following features (1) to (9) alone or in combination.

(1) A catalyst for glycerin dehydration comprising a carrier, wherein the carrier includes at least one metal oxide selected from the group consisting $TiO_2$, $SiO_2$, $Al_2O_3$, $ZrO_2$, and $Nb_2O_5$, wherein the carrier includes a bimodal porous structure in which the volume ratio between a pore volume of mesopores having a pore size of more than 2 nm to less than 50 nm to a pore volume of macropores having a pore size of 50 nm or more is equal to or more than 0.5, and wherein the carrier configures to support a W-containing metal oxide thereon or a metal oxide containing at least one element selected from the group consisting of P, Si, Mo, and V, in addition to the W-containing metal oxide thereon, and to cause a catalytic dehydrogenation reaction with glycerin to produce unsaturated aldehyde and of unsaturated carboxylic acid.

(2) The pore volume of the carrier is 0.30 cc/g or more, measured by the mercury press-in method.

(3) The average pore diameter of the carrier is 20 nm or more, preferably 30 nm or more, measured by the mercury press-in method.

(4) The volumetric basis mode diameter of the carrier is 19 nm or more, preferably 50 nm or more, measured by the mercury press-in method.

(5) The carrier is $TiO_2$.

(6) The carrier is a mixture of $TiO_2$ and at least one metal oxide selected from the group consisting of $SiO_2$, $Al_2O_3$, and $ZrO_2$.

(7) The catalyst may further comprises an additional salt of at least one element selected from Groups 1 to 16 of the periodic table.

(8) The compound other than the carrier is represented by formula (1):

$$A_aX_bY_cZ_dO_e \qquad (1)$$

in which

A is one or more cation(s) selected from elements of Groups 1 to 16 of the periodic table,
X is P or Si,
Y is W,
Z is one or more element(s) selected from the group consisting of Ti, Cr, Mn, Fe, Co, Ni, Zn, Sn, Bi, Sb, Ce, Mg, Cs, and K,

$0 \leq a < 9$,

$o \leq b \leq 1$,

$0 < c \leq 20$,

$0 \leq d \leq 20$, and

e is a value determined by oxidation numbers of each element.

(9) A method for preparing the catalyst, comprising a calcination at a temperature of 400 °C to 900 °C.

[Advantageous Effects of Invention]

The present catalyst can be used for producing unsaturated aldehyde and of unsaturated carboxylic acid by a catalytic dehydration reaction of glycerin, and can reduce a decrease in time of the conversion ratio of glycerin and the yields of unsaturated aldehyde and of unsaturated carboxylic acid. Owing to the improvement on catalyst lifetime, the efficiencies of catalytic reaction and of regeneration cycle of catalyst can be improved, so that complicated operations can be simplified, which is very beneficial for industrial plants.

[Description of Embodiments]

The present catalyst for glycerin dehydration comprising a porous carrier, wherein the porous carrier includes at least one metal oxide selected from the group consisting $TiO_2$, $SiO_2$, $Al_2O_3$, $ZrO_2$, and $Nb_2O_5$, wherein the carrier includes a bimodal porous structure in which the volume ratio between a pore volume of mesopores having a pore size of more than 2 nm to less than 50 nm to a pore volume of macropores having a pore size of 50 nm or more is equal to or more than 0.5, and wherein the porous carrier configures to support a W-containing metal oxide thereon, or a metal oxide containing at least one element selected from the group consisting of P, Si, Mo, and V, in addition to the W-containing metal oxide thereon.

Physical properties of pores, such as a pore diameter and a pore volume of the present porous carrier, are determined by the mercury press-in method (mercury intrusion method) at a mercury surface tension of 480 dyn/cm with a mercury contact angle of 140 · by a mercury porosimetry ("Pore Master 60-GT" manufacuterd by Quanta Chrome Co.). Values or positions (nm) of mesopores and macropores shown in the present description are values of the maximum pore diameter of each pore obtained in a correlation graph having axis of ordinates of -dV/d(log d) [cc/g] which expresses how much mercury penetrate in the mercury press-in method (expressed in cc for logarithm · m for 1g of sample) and axis of abscissas of the pore diameter.

Namely, the phrases of "a pore size of more than 2 nm to less than 50 nm" and " a pore size of 50 nm or more" regarding the mesopore and macropore of the present porous carrier should mean that the value of the pore diameter of each pore is located at the peak maximal value. The mesopore may preferably be in a range of more than 2 nm to less than 50nm, and more preferably in a range of 10 to 30 nm. The macropore may preferably be 50 nm or more, more preferably in a range of 50 to 300 nm.

In the present porous carrier, the ratio of the pore volume of macropores, which are within a range of more than 2 nm to less than 50 nm, to the pore volume of mesopores, which are 50 nm or more, is 0.5 or more. This ratio is obtained by: splitting the pore volumes in the correlation graph at the minimum value (downward peak) located an intermediate position between macropores and mesopores into two groups of the mesopore volume and the macropores volume, such that the mesopore volume is defined as the pore volumes that are smaller than the minimum value, and the macropores volume is defined as the pore volumes that are larger than the minimum value; and then dividing the macropore volume by the mesopore volume to obtain the value. The ratio of the macropore volume to the mesopore is 0.5 or more, preferably 1.0 or more. By selecting the above ratio, it is possible to obtain a long life catalyst for dehydration of glycerin, so that decrease in time of the conversion of glycerin and of the yield of acrolein may be prevented, in addition that acrolein may be produced at higher yield.

A feature of the pore volume of 0.30 cc/g or more determined by the mercury press-in method is also important for one embodiment of the present invention. By using a porous carrier having the pore volume of 0.30 cc/g or more, it is possible to obtain a long life catalyst for dehydration of glycerin which permits to prevent a decrease in time of the conversion of glycerin and of the yield of acrolein.

Another feature of the average pore diameter measured by the mercury press-in method being 20 nm or more, preferably 30 nm or more, is also important for one embodiment of the present invention. If a carrier has the same cumulative pore volume but has a smaller average pore diameter of less than 20 nm, then the pore volume of macropores decreases and the pore volume of mesopores increases. Since the mesopores has a larger specific surface area, and the larger specific surface area would seem to advantageously lead to a higher conversion rate of glycerin -- but indeed the reduced volume of macropores leads to a quick deterioration of catalytic activity due to a decrease in the efficiency of material transportation of products generated in the mesopores and an acceleration of coking. Therefore, the pore volume of the mesopores is one of important factors for the catalyst lifetime in the present process, since increasing the efficiency of the material transportation and preventing decrease of catalytic life caused by coking in the mesopores which is main reaction site. However, to avoid a risk of decrease in mechanical strength caused by increase of average pore diameter, in particular of pore diameter of the macropores, the average pore size may preferably be of 20 nm to 100 nm.

In an embodiment, it is also important that the volumetric basis mode diameter of the bipore carrier measured by the mercury press-in method is 19 nm or more, preferably 50 nm or more. The term "volumetric basis mode diameter" means the maximum pore diameter among the pores into which the most volume quantity of mercury penetrate. In other words, a pore diameter which is the maximal value of the largest pore in a porous carrier is equal to or larger than 19 nm, preferably 50 nm or more. For example, in case of a bipore carrier having both mesopores and macropores, if the pore volume of macropores is higher than that of mesopores, the maximum peak of macropores is located at a pore diameter of 19 nm or more, preferably 50 nm or more. If the pore volume of mesopores is higher than that of macropores, the maximum peak of mesopores is located at a pore diameter of 19 nm or more, preferably 50 nm or more. For the present porous carrier, the former is preferable. In other words, it is preferable that the ratio in pore volume of the macropores to the mesopores is equal to or higher than 1.0, and that the volumetric basis mode diameter is 19 nm or more, preferably 50 nm or more.

In another preferred embodiment, the BET specific surface area of the porous carrier measured by BET method of nitrogen adsorption is 10 to 1000 $m^2$/g, preferably 20 to 500 $m^2$/g, and more preferably 30 to 200 $m^2$/g. Increase of the specific surface may enhance higher dispersion of supported catalyst to improve activation. However, in case of the dehydration reaction of glycerin, since the reactivity of glycerin is very high and carrier itself often possesses activity, so that the carrier itself proceeds successive reactions and parallel reactions. To prevent the successive and parallel reactions., it is necessary to increase an amount of catalyst. Therefore, the preferable specific surface is particularly 30 to 100 $m^2$/g.

The catalyst for producing unsaturated aldehyde and unsaturated carboxylic acid by the dehydration of the glycerin according to the present embodiments is characterized in that it contains at least one metal oxide selected from the group consisting of $TiO_2$, $SiO_2$, $Al_2O_3$, $ZrO_2$, and $Nb_2O_5$. And hence, the catalyst may be supported on one carrier selected from the group above, or on a carrier consisting of a mixture of two or more of the metal oxides. Among them, $TiO_2$ may preferably be as an indispensable member of carrier. In case of a mixture, it may preferable be to mix $TiO_2$ with at least one metal oxide selected from the group consisting of $SiO_2$, $Al_2O_3$, $ZrO_2$, and $Nb_2O_5$. The amount of the catalyst may preferably be 1 to 90 % by weight, and more preferably 3 to 60 % by weight, to the total weight of W-containing metal oxide and carrier.

The present catalyst and carrier may have any shape, and may be in the form of, but not limited to, granule, powder, pellets, rings, trilobes, or quadrilobes. In case of for gas phase reaction, the catalyst or carrier may be molded into a shape of sphere, cylinder, trilobe, hollow trilobe, quadrilobe, hollow quadrilobe, or bar, by using a molding aid if necessary. It may also be possible to shape the catalyst together with a carrier and optionally a molding aid into any of the shapes above. If the catalyst is in a shape of trilobe or quadrilobe, the molded catalyst may have a larger available dimension to reduce a pressure fall during a reaction. The size of molded catalyst may be, in the case of sphere, 1 to 10 mm for a fixed bed catalyst, or less than 1mm for a fluidized bed catalyst.

The present catalyst for glycerin dehydration is used to dehydrate glycerin to produce unsaturated aldehyde and unsaturated carboxylic acid, and contains a W-containing metal oxide and optionally one or more other metal oxide(s) of at least one element selected from P, Si, Mo and V, supported on a porous carrier.

The present dehydrate catalyst is used for producing unsaturated aldehyde and unsaturated carboxylic acid by the dehydration of glycerin, and the metal oxide other than the carrier in the catalyst may be a compound of formula (1):

$$A_aX_bY_cZ_dO_e \qquad (1)$$

in which A is one or more cation(s) selected from elements of Groups 1 to 16 of the periodic table, X is P or Si, Y is W, Z is one or more element(s) selected from Mo and V, $0 \leq a < 9$, $0 \leq b \leq 1$, $0 < c \leq 20$, $0 \leq d \leq 20$, and e is a value determined by oxidation numbers of each element.

Source of tungsten in the W-containing metal oxide as a component to be supported on the porous carrier is not particularly limited, and may be selected from, but not limited to, any material such as ammonium paratungstate, ammonium metatungstate, tungstic acid, tungsten oxide, tungsten chloride, and organotungsten compounds e.g. tungsten ethoxide and hexacarbonyl tungsten.

The present dehydrate catalyst is used for producing unsaturated aldehyde and unsaturated carboxylic acid by the dehydration of glycerin, and the porous carrier may support thereon one or more other metal oxide(s) of at least one element selected from P, Si, Mo, and V, in addition to the W-containing metal oxide. In this case, a source material of the metal oxide may preferably be heteropolyacid(s) including the above element(s).

Here, the heteropolyacid is explained briefly. Ions of tungsten and molybdenum become oxoacids in water and the resulting oxoacid is polymerized to form high molecular polyoxoacid. In this case, not only the same kind of oxoacids are polymerized but also surrounding other oxoacid or oxoacids also are polymerized, resulting in formation of a polyacid consisting of more than two oxoacids, which is called "heteropolyacid" having a polynuclear structure. An atom that forms a center oxoacid is called as "hetero-atom", while atoms forming oxoacids surrounding the center oxoacid and forming oxoacid is called as "poly-atoms". The hetero-atom may be silicon, phosphorus, arsenic, sulfur, iron, cobalt, boron, aluminum, germanium, titanium, zirconium, cerium, and chromium. Among them, phosphorus and silicon may be preferable. The poly-atoms may be tungsten, molybdenum, vanadium, niobium, and tantalum. In the present embodiments, at least tungsten is always contained. Therefore, preferred heteropolyacid used in the glycerin dehydration is tungstophosphoric acid or tungstosilicic acid. The heteropolyacid may be a mixed coordinate type comprising phosphorus or silicon as the hetero-atom, and tungsten and molybdenum or tungsten and vanadium as the poly-atoms. It is known that the heteropolyacid may have different structures of Keggin type, Dawson type and Anderson type. The heteropolyacid may posses generally such high molecular weight as 700 to 8,500. There is a dimmer thereof, which is included in the present invention.

The present dehydrate catalyst is used for producing unsaturated aldehyde and unsaturated carboxylic acid by the dehydration of glycerin, and may include a porous carrier containing at least one metal oxide selected from the group consisting $TiO_2$, $SiO_2$, $Al_2O_3$, $ZrO_2$, and $Nb_2O_5$; a W-containing metal oxide and optionally one or more other metal oxide(s) of at least one element selected from P, Si, Mo, and V supported on the carrier; and one or more cation(s) selected from elements of Groups 1 to 16 of the periodic table therein.

The cations belonging to Group 1 to Group 16 of the Periodic Table may also include its acid salt and acid onium salts. The acid salt may be salts of sodium, potassium, rubidium, cesium, magnesium, calcium, strontium, barium, scandium, yttrium, lanthanide, titanium, zirconium, hafnium, chromium, manganese, rhenium, iron, ruthenium, osmium, cobalt, nickel, palladium, platinum, copper, silver, gold, zinc, gallium, thallium, germanium, tin, lead, bismuth, and tellurium. The acid onium salts may be amine salt, ammonium salt, phosphonium salt and sulfonium salt. Sources of the acid salt and the acid onium salts may be, but not limited to, nitrate, carbonate, sulfate, acetate, oxides, and halide of the metals or oniums. The amount of added metals or onium salt may be 0.01 to 60 % by weight, preferably 0.01 to 30 % by weight, with respect to W and one or more optional element selected from P, Si, Mo and V in addition to W.

The catalyst may contain at least one metal oxide selected from a group comprising $TiO_2$, $SiO_2$, $Al_2O_3$, $ZrO_2$, and $Nb_2O_5$ supporting W-containing metal oxide and optional at least one metal oxide of element selected from P, Si, Mo and V in addition to W-containing metal oxide. The catalyst above may be prepared by any known technique such as impregnation method, and its raw material may be nitrate, ammonium salt, hydroxide, oxide, or acid of each metal element which constitutes the active components of the catalyst. A catalyst supporting a W-containing metal oxide may also be prepared by any known technique. In practice, an aqueous solution containing compounds which contain W and optional element selected from P, Si, Mo, and V is prepared firstly. If you use heteropolyacid, an aqueous solution thereof is firstly prepared. Alternatively, their aqueous solutions may be prepared after water contained in the heteropolyacid in a form of adsorptive water and crystal water is removed partially or totally under vacuum or heat-drying. The resulting aqueous solution of W-containing metal oxide is added with a carrier containing at least one metal oxide selected from $TiO_2$, $SiO_2$, $Al_2O_3$, $ZrO_2$, and $Nb_2O_5$. Optionally, an aqueous solution containing a compound(s) containing halide, carbonate, acetate, nitrate, oxalate, phosphate, sulfate, hydroxide of metal or onium of element belonging to Group 1 to Group 16 of the Periodic Table of Elements may be added. The resulting mixture is subjected to filtration or drying under reduced pressure to obtain a solid which is then calcinated finally. Alternatively, an addition of the salts of metal or onium of element belonging to Group 1 to Group 16 of the Periodic Table may be carried out after, before, or during the addition of the aqueous solution containing W and optionally element(s) selected from P, Si, Mo, and V to the porous carrier. The calcination and drying may be carried out successively for each addition of catalytic component to carrier, or may be done after all catalytic components are added.

In one embodiment, a carrier may be contacted with a solution of a catalytic active agent, and the resulting solid content may be subjected under one or more drying-and-calcinating cycle(s). The contacting process may be carried out by the pore volume impregnation or excess solution impregnation process. The calcination, as a pre-treatment for glycerin dehydration, may be carried out in air or under inert gas such as nitrogen, helium, and argon, or under an atmosphere of a mixed gas of air and the inert gas(es). The calcination may preferably be carried out in air atmosphere which facilitates operations. A furnace for the calcination may be, but not limited to, a muffle furnace, rotary kiln, or fluidized bed furnace. The calcination may be effected even in a reaction tube which is used for the glycerin dehydration reaction.

In an embodiment, the firing temperature of 400 to 900°C may also be important. Indeed in the glycerin dehydration reaction for producing unsaturated aldehyde and unsaturated carboxylic acid, a formation or precipitation of cokes per unit time is extremely large, comparing to an oxidation of propylene and of propane, so that the life of catalyst is short, resulting in that much frequent regeneration of catalyst is required. The regeneration may be carried out by a circulation of an oxygen-containing gas which generates a large quantity of heat to remove cokes by burning. Sufficient burn and removal of cokes may not be expected at a lower temperature. In any way, the catalyst would be exposed after all to a high temperature during reaction and regeneration stage even if the calcination of the catalyst is effected at a low temperature. Therefore, in case of a catalyst which had been fired at a low temperature, there may be a difference in performance between at an initiation of reaction and after regeneration. In order to obtain the same or constant performance after several reaction and regeneration cycles as at the initial reaction, and hence to facilitate operations in reaction and regeneration, it may be advantageous to effect a calcination at a higher temperature. In case of heteropolyacid such as phosphotungstic acid and tungstosilicic acid, a decomposition of their structures starts at about 350°C and the structure of heteropolyacid will be lost when it is heated above the temperature. In case of phosphotungstic acid, it will be converted into oxides of phosphorus and tungsten or into a complex oxide other than heteropolyacid. Use of heteropolyacid such as phosphotungstic acid as a raw material of W may be suitable, but it would not have the structure of heteropolyacid because the firing temperature of 400 to 900°C, which is one of features of the present embodiment. The firing temperature may preferably be 450 to 800°C and the firing time duration may preferably be 0.5 to 10 hours.

The dehydration reaction of glycerin may be effected in gas phase or in liquid phase, and gas phase may be preferable. The gas phase reaction may be carried out in a variety of reactors such as a fixed bed, fluidized bed, circulating fluidized bed, and movable bed. Among them, the fixed bed may be preferred. The catalyst may be separated from the reactor when the catalyst is to be regenerated. If the catalyst is regenerated outside the system, the catalyst is taken out of the system and may be burnt in air or oxygen-containing gas. In case of a liquid phase reaction, the reaction may be effected in a usual vessel for a solid catalyst liquid phase reaction. Because a difference in boiling point between glycerin (290°C) and unsaturated aldehyde and unsaturated carboxylic acid is big, it is desirable to operate at a relatively lower temperature, so that acrolein produced may be distilled out continuously.

In the present glycerin dehydration reaction at gas phase for producing unsaturated aldehyde and unsaturated carboxylic acid, a reaction temperature may preferably be from 200°C to 450°C. Due to the high boiling point of glycerin, if the reaction temperature is less than 200 °C, glycerin and reaction products will be polymerized and carbonized such that the life of catalyst will shorten. On the contrary, if the reaction is effected above 450°C, parallel and successive reactions will increase such that the selectivity of unsaturated aldehyde and unsaturated carboxylic acid become lower. The reaction temperature may preferably be 250°C to 350°C.

The pressure is not limited especially, but may preferably be 10 atm or less, more preferably 5 atm or less, as the absolute pressure. Under a higher pressure, glycerin may be liquefied again. In addition, a deposition of carbon is promoted under a higher pressure, such that the life of catalyst will shorten.

A feed rate of source gas to the catalyst may preferably be 500 $h^{-1}$ to 10,000 $h^{-1}$ in term of space velocity GHSV. If the rate is lower than 500 $h^{-1}$, the selectivity will be lowered by successive reactions. If the rate exceeds 10,000 $h^{-1}$, the conversion efficiency will be lowered.

In case of liquid phase reaction, the temperature may preferably be from 150°C to 350°C. At a lower temperature, the selectivity is improved but the conversion is lowered. The pressure is not limited especially but it may be pressurized from 3 atm to 70 atm if necessary.

Glycerin as a raw material may be commercially available in a form of an aqueous solution. A concentration of the glycerin aqueous solution may preferably be from 5 % to 90 % by weight, preferably from 10 to 60 % by weight. If the glycerin concentration is too high, undesirable glycerin ether will be formed, or glycerin will undesirably react with the resulting unsaturated aldehyde and unsaturated carboxylic acid. In addition, too much glycerin will require enormous energy to gasify.

The concentration of glycerin in a mixed gas which is fed to the present catalyst may be 1 to 30 mol %, preferably 1 to 12 mol %, and more preferably 3 to 10 mol %.

The present glycerin dehydration reaction is carried out in the presence of an oxygen-containing gas such as oxygen or air. The concentration of oxygen may be 1 to 10 mol %, preferably 2 to 7 mol %.

[Examples]

The present invention will be explained in more details by using several Examples. The present invention is however not limited to the following Examples but covers those that are within the spirit of the present invention. In the Examples and Comparative Examples, the unit of "%" means mole %.

<Example 1>

Catalyst of PW/TiO$_2$ for dehydration reaction of glycerin was prepared as following. 13.2 g of phosphotungstic acid (Nippon Inorganic Color & Chemical Co., Ltd.) was dissolved in 100 ml of pure water. Separately, 100g of anatase TiO$_2$ pellets ("ST 31119" manufactured by Saint-Gobain; diameter of 3.2 mm · length of 5 mm; 48.2 m$^2$/g; 0.36 cc/g; pore volume ratio = 1.4) was placed on a porcelain dish onto which the above aqueous solution of phosphotungstic acid was added and left for 2 hours. The mixture was dried up at 120°C for 10 hours, and then calcinated in an air atmosphere at 500 °C for 3 hours.

The resulted catalyst was subjected to an evaluation test in a pass-through type fix bed reactor operated at ambient pressure. 30 cc of the catalyst was packed in a SUS reaction tube (20 mm diameter). An aqueous solution of 50 wt % of glycerin was pre-heated in a vaporizer heated at 330 °C and the resulted gasified glycerin was pumped and fed directly to the catalyst together with air flow (flow rate: 23.4 g/hr). The reactor containing the catalyst was heated to 290 °C. The feed stream had following composition: (glycerin : oxygen : nitrogen : water) = (4.7 mol % : 2.8 mol % : 68.5 mol % : 24.0 mol %). The GHSV was 2,020 $h^{-1}$. The resulted products were collected in a condenser as condensates, and the quantitative determination of all components was carried out by a gas chromatograph ("7890A" manufactured by Agilent; DB-WAX column). Factor of each product was corrected by the gas chromatograph to obtain absolute contents of the fed glycerin, remained glycerin, and products. The conversion of raw material (glycerin conversion), the selectivity of objective substance (acrolein selectivity), and the yield of objective substance (acrolein yield) were calculated by following equations:

$$\text{The conversion of raw material (\%)} = \{(\text{a mole number of material reacted}) / (\text{a mole number of material fed})\} \cdot 100$$

$$\text{The selectivity of objective substance (\%)} = \{(\text{a mole number of objective substance obtained}) / (\text{a mole number of material reacted})\} \cdot 100$$

$$\text{The yield of objective substance (\%)} = \{(\text{a mole number of objective substance obtained}) / (\text{a mole number of material fed})\} \cdot 100$$

The quantitative analysis was carried out in every several hours. The glycerin conversion and the acrolein yield were compared for each catalyst at a reaction time of 19 hours and 43 hours.

<Example 2>

The same experimental procedure and estimation as Example 1 were carried out, except that anatase $TiO_2$ pellet ("ST 31119" manufactured by Saint-Gobain; diameter of 3.2 mm · length of 5 mm; 29.2 $m^2$/g; 0.30 cc/g; pore volume ratio = 1.5) was used as $TiO_2$. The result is shown in Table 1.

<Example 3>

The same experimental procedure and estimation as Example 1 were carried out, except that anatase $TiO_2$ pellet ("ST 31119" manufactured by Saint-Gobain; diameter of 3.2 mm · length of 5 mm; 35.6 $m^2$/g; 0.33 cc/g; pore volume ratio = 1.1) was used as $TiO_2$. The result is shown in Table 1.

The cumulative pore volume, the ratio in pore volume, and the average pore diameter of Examples 2 and 3 are within a range of this invention. The results reveal that these catalysts show high glycerin conversion and high acrolein yield and reduced decrease of the glycerin conversion and of the acrolein yield, in spite of relatively lower specific surface area, comparing to Comparative Examples.

<Example 4>

The same experimental procedure and estimation as Example 1 were carried out, except that anatase $TiO_2$ pellet ("ST 31119" manufactured by Saint-Gobain; diameter of 3.2 mm · length of 5 mm; 39.9 $m^2$/g; 0.36 cc/g; pore volume ratio = 1.3) was used as $TiO_2$. The result is shown in Table 1.

<Example 5>

The same experimental procedure and estimation as Example 1 were carried out, except that anatase $TiO_2$

pellet ("ST 31119" manufactured by Saint-Gobain; diameter of 3.2 mm · length of 5 mm; 45.6 $m^2/g$; 0.36 cc/g; pore volume ratio = 1.4) was used as $TiO_2$. The result is shown in Table 1.

<Example 6>

Catalyst of $W/TiO_2$ for dehydration reaction of glycerin was prepared as following. 12.2 g of ammonium metatungustate (Nippon Inorganic Color & Chemical Co., Ltd.) was dissolved in 100 ml of pure water. Separately, 100g of anatase $TiO_2$ pellets ("ST 31119" manufactured by Saint-Gobain; diameter of 3.2 mm length of 5 mm; 48.2 $m^2/g$; 0.36 cc/g; pore volume ratio = 1.4) was placed on a porcelain dish onto which the above aqueous solution of ammonium metatungustate was added and left for 2 hours. The mixture was dried up at 120°C for 10 hours, and then calcinated in an air atmosphere at 500 °C for 3 hours.

<Example 7>

The same operation as Example 1 was carried out, except that anatase $TiO_2$ ring ("ST 31119" manufactured by Saint-Gobain; diameter of 4.5 mm · length of 5 mm; 54 $m^2/g$; 0.32 cc/g; pore volume ratio = 0.5) was used as $TiO_2$.

<Example 8>

The same operation as Example 1 was carried out, except that anatase $TiO_2$ trilobe ("ST 31119" manufactured by Saint-Gobain; diameter of 4.5 mm · length of 5 mm; 53 $m^2/g$; 0.40 cc/g; pore volume ratio = 0.9) was used as $TriO_2$.

<Example 9>

The same operation as Example 1 was carried out, except that anatase $TiO_2$ quadrilobe ("ST 31119" manu-factured by Saint-Gobain; diameter of 4.5 mm · length of 5 mm; 54 $m^2/g$; 0.38 cc/g; pore volume ratio = 0.9) was used as $TiO_2$. The same reaction estimation as Example 1 was carried out. The result is shown in Table 1.

<Comparative Example 1>

In the Comparative Examples, a sample of $TiO_2$ which is outside the scope of present invention was prepared and evaluated as following.
The same experimental procedure and estimation as Example 1 were carried out, except that anatase $TiO_2$ sphere ("CS-300S" manufactured by Sakai Kagaku; diameter: 3.0 mm; 75 $m^2/g$; 0.40 cc/g; pore volume ratio = 0.4) was used as $TiO_2$. The result is shown in Table 1.

<Comparative Example 2>

The same experimental procedure and estimation as Example 1 were carried out, except that anatase $TiO_2$ sphere ("CS-300S" manufactured by Sakai Kagaku; diameter: 3.0 mm; 52 $m^2/g$; 0.32 cc/g; pore volume ratio = 0.3) was used as $TiO_2$. The result is shown in Table 1.
Catalysts of Comparative Examples 1 and 2 had the same or higher pore volume and specific surface area as Examples 1, 4, and 5, but the catalytic activity of Comparative Example 1 decreased much rapidly (comparison in the glycerin conversion and the acrolein yield at 19 hours and 43 hours). Comparative Example 1 has the ratio in pore volume of the macropores to the mesopores is 0.4 which is outside a requirement of higher than 0.5 of the present invention. The results reveal that the life of catalyst is influenced by the ratio of pore volumes.

<Comparative Example 3>

The same operation as Example 1 was carried out, except that anatase $TiO_2$ pellet ("ST 31119" manufactured by Saint-Gobain; diameter of 3.2 mm · length of 5 mm; 42.4 $m^2/g$; 0.26 cc/g; pore volume ratio = 1.6) was used as $TiO_2$. The same reaction estimation as Example 1 was carried out. The result is shown in Table 1.
The catalyst of Comparative Example 3 has similar specific surface area, average pore diameter, and pore volume as Examples 1 and 5, but the pore volume of 0.26 cc/g is outside the claimed range (0.30 cc/g or more). The result reveals that deteriorations in time of the glycerin conversion and of the acrolein yield are also influenced by the cumulative pore volume in addition to the specific surface, average pore diameter, and the ratio of pore volumes.

<Comparative Example 4>

In Example 6, the same experimental procedure and estimation as Example 1 was carried out, except that

anatase $TiO_2$ pellet ("ST 31119" manufactured by Saint-Gobain; diameter of 3.2 mm · length of 5 mm; 42.4 $m^2/g$; 0.26 cc/g; pore volume ratio = 1.6) was used as $TiO_2$. The same reaction estimation was carried out. The result is shown in Table 1.

By comparing Comparative Example 4 and Example 6, difference in physical properties of $TiO_2$ when the carrier supports a metal oxide consisting of tungsten oxide alone can be evaluated. A comparative carrier of Comparative Example 4 has same specific surface area, average pore diameter, and the ratio in pore volumes as Example 6, but the pore volume of 0.26 cc/g is outside the claimed range (0.30 cc/g or more), as similar to $PW/TiO_2$ of Comparative Example 3. The result reveals that deteriorations in time of the glycerin conversion and of the acrolein yield are also influenced by the ranges of physical properties in the case that a porous carrier supports a metal oxide consisting of tungsten oxide alone.

[Table 1]

| Catalyst # | pore volume (cc/g) | ratio of pore volumes of macropore/mesopore (-) | average pore diameter (nm) | maxium pore diameter of mesopores (nm) | maxium pore diameter of macropores (nm) | volumetric basis mode diameter (nm) | specific surface area (m2/g) | reaction time (hr) | conversion of glycerin (%) | yield of acrolein (%) |
|---|---|---|---|---|---|---|---|---|---|---|
| Example 1 | 0.36 | 1.4 | 37.5 | 18.5 | 130 | 130 | 48.2 | 19 | 99.6 | 77.0 |
|  |  |  |  |  |  |  |  | 43 | 95.6 | 73.6 |
| Example 2 | 0.30 | 1.5 | 47.9 | 22.2 | 149 | 149 | 29.2 | 19 | 97.9 | 76.7 |
|  |  |  |  |  |  |  |  | 43 | 88.4 | 67.8 |
| Example 3 | 0.33 | 1.1 | 36.6 | 20.7 | 136 | 136 | 35.6 | 19 | 98.2 | 75.0 |
|  |  |  |  |  |  |  |  | 43 | 89.4 | 67.0 |
| Example 4 | 0.36 | 1.3 | 39.7 | 19.9 | 160 | 160 | 39.9 | 19 | 99.5 | 77.4 |
|  |  |  |  |  |  |  |  | 43 | 93.2 | 70.9 |
| Example 5 | 0.36 | 1.4 | 37.6 | 18.4 | 159 | 159 | 45.6 | 19 | 99.7 | 76.9 |
|  |  |  |  |  |  |  |  | 43 | 94.8 | 72.1 |
| Example 6 | 0.36 | 1.4 | 37.5 | 18.5 | 130 | 130 | 48.2 | 19 | 99.6 | 74.3 |
|  |  |  |  |  |  |  |  | 43 | 93.6 | 68.3 |
| Example 7 | 0.32 | 0.5 | 22.6 | 19.9 | 83 | 20 | 54 | 19 | 99.6 | 74.2 |
|  |  |  |  |  |  |  |  | 43 | 93.6 | 71.6 |
| Example 8 | 0.40 | 0.9 | 44.1 | 20.5 | 134 | 20 | 53 | 19 | 99.4 | 74.5 |
|  |  |  |  |  |  |  |  | 43 | 94.7 | 69.4 |
| Example 9 | 0.38 | 0.9 | 34.1 | 19.9 | 127 | 20 | 54 | 19 | 98.8 | 72.8 |
|  |  |  |  |  |  |  |  | 43 | 91.4 | 65.2 |
| comp.ex. 1 | 0.40 | 0.4 | 23.2 | 16.6 | 452 | 16.6 | 75.0 | 19 | 99.3 | 73.5 |
|  |  |  |  |  |  |  |  | 43 | 88.2 | 64.8 |
| comp.ex 2 | 0.32 | 0.3 | 23.9 | 18.9 | 140 | 18.9 | 52.0 | 19 | 96.8 | 70.4 |
|  |  |  |  |  |  |  |  | 43 | 78.0 | 56.2 |

| Catalyst # | pore volume (cc/g) | ratio of pore volumes of macropore/mesopore (-) | average pore diameter (nm) | maxium pore diameter of mesopores (nm) | maxium pore diameter of macropores (nm) | volumetric basis mode diameter (nm) | specific surface area (m2/g) | reaction time (hr) | conversion of glycerin (%) | yield of acrolein (%) |
|---|---|---|---|---|---|---|---|---|---|---|
| comp. ex 3 | 0.26 | 1.6 | 33.9 | 14.8 | 155 | 155 | 42.4 | 19 | 96.3 | 76.0 |
|  |  |  |  |  |  |  |  | 43 | 83.1 | 64.8 |
| comp.ex. 4 | 0.26 | 1.6 | 33.9 | 14.8 | 155 | 155 | 42.4 | 19 | 95.4 | 73.4 |
|  |  |  |  |  |  |  |  | 43 | 78.9 | 58.9 |

EP 2 737 943 A2

The catalyst of Example 7 was also evaluated by the following condition. The feed stream had following composition: (glycerin : oxygen : nitrogen : water) = (6.0 mol % : 2.0 mol % : 61.3 mol % : 30.7 mol %). The GHSV was 2,020 $h^{-1}$. The yield of acrolein at 19 hours was 73.5 %.

From the results of Examples and Comparative Examples, following conclusions are obtained:

(1) The present catalyst for glycerin dehydration comprises a porous carrier, wherein the porous carrier includes at least one metal oxide selected from the group consisting $TiO_2$, $SiO_2$, $Al_2O_3$, $ZrO_2$, and $Nb_2O_5$, wherein the carrier includes a bimodal porous structure in which the volume ratio between a pore volume of mesopores having a pore size of more than 2 nm to less than 50 nm to a pore volume of macropores having a pore size of 50 nm or more is equal to or more than 0.5, and wherein the porous carrier configures to support a W-containing metal oxide thereon, or a metal oxide containing at least one element selected from the group consisting of P, Si, Mo, and V, in addition to the W-containing metal oxide thereon. There is no substantial difference in glycerin conversion and in acrolein yield after the reaction time of 19 hours and of 43 hours, so that the life of present catalyst should be improved

(2) A conventional catalyst in which a carrier is a porous support like bipore and has a pore volume of 0.3 cc/g or more, cannot prevent a decrease in time of glycerin conversion and of acrolein yield, and cannot have an improved lifetime. In addition, its mechanical strength is lost if the pore volume increases. Advantage of the higher pore volume and hence longer catalyst life may be obtained only when all requirement of the ratio in pore volume of macropores to the mesopores, the average pore diameter, and the volumetric basis mode diameter according to the present invention are satisfied, resulting in that the life of catalyst is improved.

(3) In other words, the present catalyst may improve a decease of catalytic activity in time or the life of catalyst without spoiling the glycerin conversion and the acrolein yields, comparing to a conventional $TiO_2$ having physical properties outside the present invention. The present catalyst above comprises a porous carrier, wherein the porous carrier includes at least one metal oxide selected from the group consisting $TiO_2$, $SiO_2$, $Al_2O_3$, $ZrO_2$, and $Nb_2O_5$, wherein the carrier includes a bimodal porous structure in which the volume ratio between a pore volume of mesopores having a pore size of more than 2 nm to less than 50 nm to a pore volume of macropores having a pore size of 50 nm or more is equal to or more than 0.5, in particular the average pore diameter of the carrier being 20 nm or more, more preferably 30 nm or more, and the volumetric basis mode diameter of the carrier being 19 nm or more, preferably 50 nm or more, and wherein the porous carrier configures to support a W-containing metal oxide thereon, or a metal oxide containing P in addition to the W-containing metal oxide thereon.

**Claims**

1. A catalyst for glycerin dehydration comprising a carrier,
wherein the carrier includes at least one metal oxide selected from the group consisting $TiO_2$, $SiO_2$, $Al_2O_3$, $ZrO_2$, and $Nb_2O_5$,
wherein the carrier includes a bimodal porous structure in which the volume ratio between a pore volume of mesopores having a pore size of more than 2 nm to less than 50 nm to a pore volume of macropores having a pore size of 50 nm or more is equal to or more than 0.5, and
wherein the carrier configures to support a W-containing metal oxide thereon and to cause a catalytic dehydrogenation reaction with glycerin to produce acrolein and acrylic acid.

2. The catalyst of claim 1, wherein the carrier further supports a metal oxide containing at least one element selected from the group consisting of P, Si, Mo, and V, in addition to the W-containing metal oxide.

3. The catalyst of claim 1 or 2, wherein the pore volume of the carrier is 0.30 cc/g or more, measured by the mercury press-in method.

4. The catalyst of any one of claims 1 to 3, wherein the average pore diameter of the carrier is 20 nm or more, measured by the mercury press-in method.

5. The catalyst of any one of claims 1 to 3, wherein the average pore diameter of the carrier is 30 nm or more, measured by the mercury press-in method.

6. The catalyst of any one of claims 1 to 5, wherein the volumetric basis mode diameter of the carrier is 19 nm or more, measured by the mercury press-in method.

7. The catalyst of any one of claims 1 to 5, wherein the volumetric basis mode diameter of the carrier is 50 nm or more, measured by the mercury press-in method.

8. The catalyst of any one of claims 1 to 7, comprising a carrier of $TiO_2$ mixed with at least one metal oxide selected from the group consisting of $SiO_2$, $Al_2O_3$, $ZrO_2$, and $Nb_2O_5$.

9. The catalyst of any one of claims 1 to 8, further comprising an additional salt of at least one element selected from Groups 1 to 16 of the periodic table.

10. The catalyst of any one of claims 1 to 9, wherein the metal oxide other than the carrier in the catalyst is a compound of formula (1):

$$A_aX_bY_cZ_d4_e \qquad (1)$$

in which

A is one or more cation(s) selected from elements of Groups 1 to 16 of the periodic table,
X is P or Si,
Y is W,
Z is one or more element(s) selected from Mo and V,
$0 \leq a < 9$,
$0 \leq b \leq 1$,
$0 < c \leq 20$,
$0 \leq d \leq 20$, and
e is a value determined by oxidation numbers of each element.

11. The catalyst of any one of claims 1 to 10, wherein the amount of the metal oxide of formula (1) supported on the carrier is 1 to 90 wt%.

12. A method for preparing a catalyst used in a glycerin dehydration for producing acrolein and acrylic acid by a catalytic dehydration reaction of glycerin, the method comprising the steps of:

adding a carrier into a solution containing a metal oxide represented by the above formula (1) or its salt, the carrier comprising at least one metal oxide(s) selected from the group consisting of $TiO_2$, $SiO_2$, $Al_2O_3$, $ZrO_2$, and $Nb_2O_5$;
drying the mixture to obtain a solid substance; and
calcinating the resulted solid substance.

13. The method of claim 12, wherein the calcinating step is carried out under an atmosphere of air, an inert gas, a mixed gas of oxygen and inert gas, or a reducing gas of hydrogen and inert gas.

14. The method of claim 12 or 13, wherein the calcinating step is carried out at a temperature of 400 °C to 900 °C for 0.5 to 10 hours.

15. Use of the supported catalyst of any one of claims 1 to 11, in a production of acrolein and acrylic acid by a catalytic dehydration reaction of glycerin.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5387720 A
- FR 695931
- WO 2006087084 A
- WO 2009044081 A
- WO 2009128555 A
- WO 2010046227 A
- WO 2007058221 A
- JP 2008088149 A
- JP 2010253374 A
- JP 2007301506 A